(19) Europäisches Patentamt
European Patent Office
Office européen des brevets

(11) Numéro de publication: **0 435 709 A1**

# (12) DEMANDE DE BREVET EUROPEEN

(21) Numéro de dépôt: 90403330.5

(51) Int. Cl.5: **C07H 17/04**

(22) Date de dépôt: 23.11.90

(30) Priorité: 24.11.89 FR 8915483

(43) Date de publication de la demande:
03.07.91 Bulletin 91/27

(84) Etats contractants désignés:
AT BE CH DE DK ES FR GB GR IT LI LU NL SE

(71) Demandeur: PIERRE FABRE MEDICAMENT
125, Rue de la Faisanderie
F-75116 Paris(FR)

(72) Inventeur: Robin, Jean-Pierre
c/0, Seripharm, 28 rue Sainte Croix
F-72000 Le Mans(FR)
Inventeur: Houlbert, Nadine
c/0, Seripharm, 28 rue Sainte Croix
F- Le Mans(FR)
Inventeur: Lenain, Valéry
c/0, Seripharm, 28 rue Sainte Croix
F- 72000 Le Mans(FR)

(74) Mandataire: Ahner, Francis et al
CABINET REGIMBEAU, 26, avenue Kléber
F-75116 Paris(FR)

## (54) Dérivés d'étoposide, leur préparation et leur application comme intermédiaires de synthèse.

(57) La présente invention concerne les tétra-O-acétyl-β-D-O-glucosides de déméthyl-4′ alcoxy-carbonyl-4′ épipodophyllotoxines répondant à la formule générale III

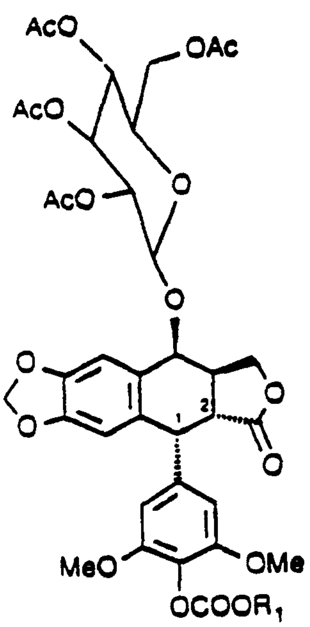

(III)

dans laquelle :
$R_1$ represente un radical alcoyle inférieur en $C_1$ à $C_4$, linéaire ou ramifié et éventuellement substitué par un ou plusieurs atomes d'halogène, en particulier de chlore.

EP 0 435 709 A1

La présente invention concerne de nouveaux tétra-O-acétyl-β-D-O-glucosides de déméthyl-4' alcoxy-carbonyl-4' épipodophyllotoxines, leur préparation ainsi que leur utilisation en tant qu'intermédiaires de synthèse nécessaires à la préparation d'étoposides qui sont des anti-cancéreux d'intérêt majeur en thérapeutique humaine.

La présente invention concerne plus particulièrement les tétra-O-acétyl-β-D-O-glucosides de déméthyl-4' alcoxy-carbonyl-4' épipodophyllotoxines répondant à la formule III

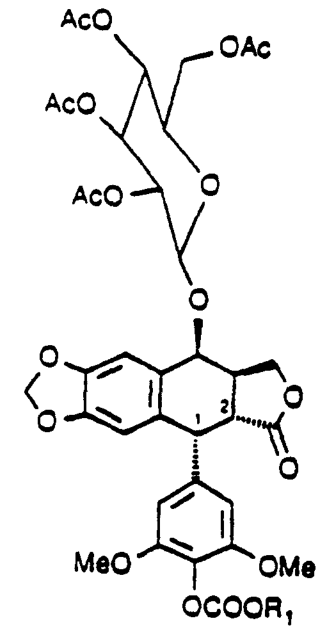

(III)

dans laquelle $R_1$ représente un radical alcoyle inférieur en $C_1$ à $C_4$, linéaire ou ramifié et éventuellement substitué par un ou plusieurs atomes d'halogène, en particulier de chlore.

La présente invention se rapporte également a un procédé pour la préparation d'un tel composé de formule générale III tel que défini précédemment, qui se caractérise par la mise en oeuvre des étapes réactionnelles successives suivantes :

i) on protège sélectivement l'hydroxyle phénolique de la déméthyl-4' épipodophyllotoxine de formule VII

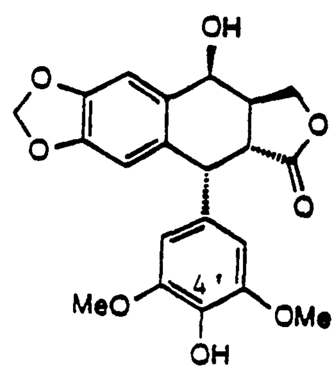

(VII)

par réaction de ce dernier composé de formule VII avec un chloroformiate d'alcoyle de formule VI

$ClCO_2-R_1$ (VI)

dans laquelle $R_1$ a la signification donnée à la revendication 1 à propos de la formule III, et

ii) on soumet l'alcoxycarbonyl-4' déméthyl-4' épipodophyllotoxine ainsi obtenue, qui répond à la formule générale V

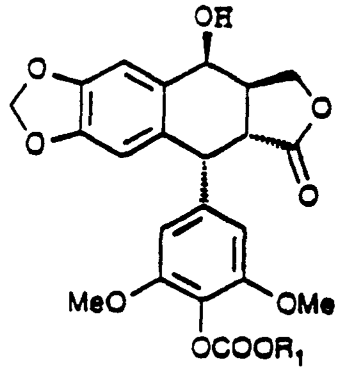

(V)

dans laquelle $R_1$ a la même signification que ci-dessus, à une réaction de couplage avec le tétra-O-acétyl-2,3,4,6, = β-D-glucose de formule IV

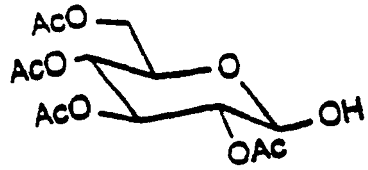

(IV)

Selon une caractéristique particulière de ce procédé, la réaction de couplage ii) est réalisée en présence d'un acide de Lewis, dans un solvant de préférence constitué par un hydrocarbure chloré. L'acide de Lewis sera par exemple de l'éthérate de trifluorure de bore ou encore le chlorure stannique. A titre de solvant réactionnel, on utilisera en particulier le dichlorométhane ou le dichloroéthane.

Contrairement aux méthodes analogues décrites jusqu'à présent **[(M. Kuhn et A. Von Wartburg, Helv. Chim. Acta, 52, 948-955 (1969)]**, cette réaction de couplage est conduite à une température comprise entre -20 et -25° C. Le fait de travailler dans cette gamme de faibles températures se traduit par une diminution importante de la proportion de l'anomère alpha indésirable répondant à la formule suivante :

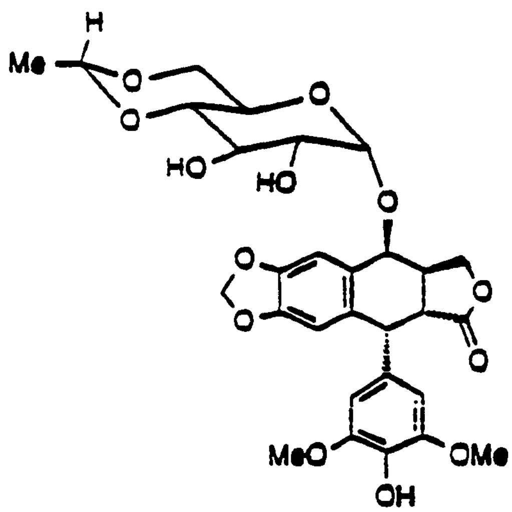

(I')

La présente invention s'applique également à l'application de ces composés de formule générale III tels que définis précédemment, à titre d'intermédiaires de synthèse utiles dans la préparation d'alkylidènes glucosides de déméthyl-4'-épipodophyllotoxine.

Ces composés dont on a déjà précisé précédemment l'intérêt anti-cancéreux majeur, sont obtenus par déprotection totale d'un composé de formule générale III, qui conduit à la β-D-glucopyranosyl-4 déméthyl-4' épipodophyllotoxine de formule II

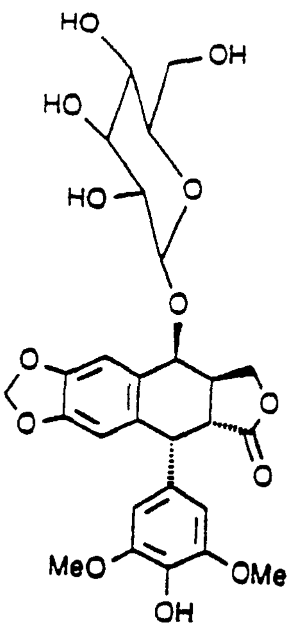

(II)

laquelle est ensuite soumise à une acétylation pour condure à un alkylidène glucoside de déméthyl-4' épipodophyllotoxine de formule générale I

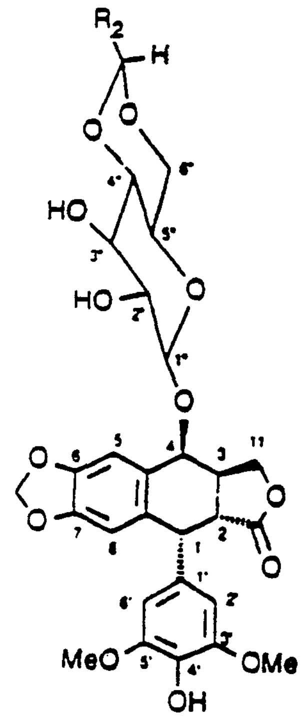

(I)

dans laquelle $R_2$ represente un radical alcoyle inférieur en $C_1$ à $C_4$, un radical aryle ou encore hétéroaryle tel que thiophényle.

On indiquera à propos de ces composés de formule I, que le dérivé particulier dans lequel $R_2$ représente le radical méthyle est connu sous le nom d'étoposide ou VP 16 **["étoposide (VP-16) - Current status and New Developments" B.F. Issell and all (1984), Academic Press, Inc].** Le composé de formule I dans laquelle $R_2$ représente le radical thiophényle est connu sous le nom de téniposide ou VM 26.

La déprotection des composés de formule générale III peut être obtenue avantageusement dans d'excellentes conditions par mise en solution dans un alcool à bas point d'ébullition, de préférence dans le méthanol ou dans un mélange du même alcool avec un co-solvant, de préférence, le tétrahydrofuranne. La réaction est alors conduite en présence de catalyseurs homogène et hétérogène combinés, par exemple un

acétate métallique, de préférence de zinc, et du zinc en poudre.

Cette réaction de déprotection totale donne en 12 à 192 heures le glucoside déprotégé sur ses deux sites, contrairement à la méthode traditionnelle qui nécessitait la mise en oeuvre de deux étapes distinctes successives.

Tel que cela sera rappelé ci-après, la fiabilité de cette méthode de synthèse a été vérifiée en soumettant un glucoside ainsi déprotégé à une réaction d'acétylation selon la méthode décrite originalement **[C. Keller-Julin, M. kuhn, A. Von Wartburg et H. Staehlin, J. Med. Chem. 14, 936-940 (1971)].**

On a ainsi pu obtenir dans d'excellentes conditions et avec un rendement optimum un acétal cyclique dont le représentant le plus significatif est le O-O'-éthylidène-4,6-β-D-O-glucoside de déméthyl-4' épipodophyllotoxine ou étoposide, c'est-à-dire un composé de formule I dans laquelle $R_2$ représente le groupe méthyle.

Avantageusement, un tel étoposide est finalement soumis à une opération de purification par recristallisation dans l'acétonitrile aqueux.

L'objet de la présente invention sera décrit ci-après à propos de quelques exemples donnés à simple titre d'illustration.

**SYNTHESE DES REACTIFS DE DEPART**

**a) Synthèse des réactifs de départ**

Le tétra-O-acétyl-2,3,4,6,-β-glucose IV est synthétisé selon les procédés décrits dans la littérature **[Organic Syntheses, Coll. Vol. 3, 11 ; Organic Syntheses, 434]**, tandis que l'aglycone est obtenu à partir de la déméthyl-4' épipodophyllotoxine VII, elle-même obtenue à l'aide de procédés dérivés de la littérature **[M. Kuhn, C. Keller-Julin et A. Von Wartburg, Helv. Chim. Acta, 52, 944-947 (1969)].** Ce dernier composé est donc synthétisé en soumettant l'aglycone à l'action d'un chloroformiate d'alcoyle convenablement substitué (exemples 1 à 4). La réaction a lieu en milieu neutre, étant donné la fragilité aux bases du composé cherché. Dans tous les cas, le chloroformiate est ajouté suffisamment lentement, pour que ce dernier soit constamment en défaut de concentration par rapport au phénol, de façon à obtenir un minimum du composé biprotégé correspondant, c'est-à-dire, également protégé en 4, ce produit étant inutilisable dans l'étape suivante. Le contrôle de la température et de l'évolution de la réaction par chromatographie sur couche mince permet d'atteindre ce but. L'excellent pouvoir cristallogène de cet ester intermédiaire rend la purification aisée.

**EXEMPLE 1**

**[(Trichloro-2,2,2) éthoxycarbonyl]-4' déméthyl-4' épipodophyllotoxine V $R_1 = CH_2CCl_3$]**

On introduit sous agitation, de la déméthyl-4' épipodophyllotoxine (1 kg, 2,5 moles), dans du dichloro-1,2 éthane sec (8 l) et de la pyridine anhydre (350 ml). Le milieu réactionnel est maintenu à 25°C, et on ajoute en 2 h, du chloroformiate de (trichloro-2,2,2) éthyle (444 ml, 1,3 eq) en solution dans du dichloro-1,2 éthane sec (1,5 l). La réaction est poursuivie 45 mn après l'addition, laquelle est éventuellement prolongée en cas de réaction incomplète (CCM). La réaction est alors bloquée par un lavage à l'acide chlorhydrique. La phase organique est lavée avec de la saumure et séchée ($MgSO_4$), puis évaporée sous pression réduite. On obtient une gelée qui est soumise telle quelle à la chromatographie sur gel de silice (Si60, 40-60 µm, MeOH (0 à 5 %)/$CH_2Cl_2$) ; M = 1,3 kg ; Rdt brut = 90%; F = 126-129°C ; $[\alpha]_D^{22} = -40°$ (c = 1 ; $CHCl_3$).

**EXEMPLE 2 :**

**Méthoxycarbonyl-4' déméthyl-4' épipodophyllotoxine V ($R_1$ = Me)**

On reprend le mode opératoire de l'exemple 1, mais en utilisant du chloroformiate de méthyle (250 ml) en lieu et place du chloroformiate de trichloroéthyle, pour obtenir le produit recherché. M = 1,13 kg ; Rdt = 99% ; F = 254-257°C ; $[\alpha]_D^{22} = -50°$ (C = 1 ; $CHCl_3$).

**EXEMPLE 3 :**

**Ethoxycarbonyl-4' déméthyl-4' épipodophyllotoxine V ($R_1$ = Et)**

On reprend le mode opératoire de l'exemple 1, mais en utilisant du chloroformiate d'éthyle (310 ml) en lieu et place du chloroformiate de trichloroéthyle, pour obtenir le produit recherché sous forme d'un composé amorphe. M = 1060 g ; Rdt = 90 %.

**EXEMPLE 4 :**

**Butoxycarbonyl-4' déméthyl-4' épipodophyllotoxine V ($R_1 = C_4H_9$)**

On reprend le mode opératoire de l'exemple 1, mais en utilisant du chloroformiate de butyle (380 ml) en lieu et place du chloroformiate de trichloroéthyle, pour obtenir le produit recherché sous forme d'un verre. M = 1060 g ; Rdt = 85%.

**EXEMPLE 5 :**

**[(Tétra-O-acétyl-2,3,4,6) -D-glucopyranosyl]-4 [(trichloro-2,2,2) éthoxycarbonyl]-4' déméthyl-4' épipodophyllotoxine III ($R_1 = CH_2CCl_3$)**

On dissout de la trichloroéthoxycarbonyl-4' épipodophyllotoxine (1300 g, 2,26 moles), puis du tétra-O-acétyl-)3-$\beta$-glucose (1180 g, 3,39 moles, 1,5 eq) dans du dichloro-1,2 éthane sec (8 l). On refroidit le mélange à -22° C, puis on ajoute en 25 mn environ, du trifluorure de bore (650 ml, 5,15 moles, 2,3 eq) en maintenant le milieu à une température de -20° C. Le milieu réactionnel est agité ensuite pendant 1 heure, puis on ajoute une solution de pyridine (450 ml) dans du dichloro-1,2 éthane (450 ml). Le mélange est alors lavé à l'aide d'une solution d'acide sulfurique. Les phases organiques séchées ($MgSO_4$), puis évaporées sous pression réduite donnent une mousse, utilisée telle quelle dans l'étape suivante. M = 1640 g ; Rdt = 80 % ; F = 128-130° C ; $[\alpha]_D^{22} = -35°$ (c = 1 ; $CHCl_3$).

**EXEMPLE 6 :**

**[(Tétra-O-acétyl-2,3,4,6) -D-glucopyranosyl]-4 méthoxycarbonyl-4' déméthyl-4' épipodophyllotoxine III ($R_1 = CH_3$)**

On reprend le mode opératoire de l'exemple 5, mais en utilisant de la méthoxycarbonyl-4' déméthyl-4' épipodophyllotoxine (1035 g) en lieu et place de la trichloroéthoxycarbonyl-4' déméthyl-4' épipodophyllotoxine, et l'on obtient le produit recherché sous forme d'un composé amorphe, M 1425 g ; Rdt = 80%.

**EXEMPLE 7 :**

**$\beta$-D-glucopyranosyl-4 déméthyl-4' épipodophyllotoxine II**

**1°) A partir du dérivé issu de l'exemple 5.**

Dans du méthanol (7 l) on introduit du glucoside protégé issu de l'exemple 5 (500 g, 0,55 mole), de la poudre de zinc (500 g), de l'acétate de zinc dihydraté (75 g) et de l'eau (45 ml). Le mélange hétérogène est porté au reflux sous agitation vigoureuse pendant 16 h. Après contrôle (C.C.M.), le mélange est filtré à chaud, puis le solide résiduel est extrait à nouveau au méthanol chaud. Les phases alcooliques sont réunies et concentrées sous pression réduite jusqu'à l'obtention d'une mousse qui est soumise à la chromatographie sur gel de silice (Si60, 40-60 $\mu$m ; MeOH (6 à 20%)/$CH_2CL_2$). Le produit obtenu cristallise à froid dans l'éther éthylique. M = 186g ; Rdt = 60 % ; F = 206-208° C ; $[\alpha]_D^{22} = -75°$ (c = 1 ; $H_2O$).

**2°) A partir du dérivé issu de l'exemple 6**

On reprend le mode opératoire de l'exemple 7, mais en utilisant du glucoside protégé issu de l'exemple 6 (435 g), en lieu et place du glucoside protégé issu de l'exemple 5. Le temps de réaction est de 192 h, pour obtenir le produit recherché sous forme d'un composé amorphe. M = 170 g ; Rdt = 55 %.

**EXEMPLE 8 :**

**[(Ethylidène-4,6)$\beta$-D-glucopyranosyl]-4 déméthyl-4' épipodophyllotixine I $R_2$ = Me)**

Dans de l'acétonitrile sec (5 l), on ajoute de 1'α-D-glucopyranosyl-4 déméthyl-4' épipodophyllotoxine (250 g, 0,44 mole), du diéthylacétal de l'acétaldéhyde distillé (1350 ml) et de l'acide paratoluène-sulfonique (25 g). Le milieu réactionnel est agité pendant 30 min. à température ambiante. Le milieu est dilué par du chloroforme (8 l), lavé à l'eau, séché ($MgSO_4$) et évaporé sous pression réduite pour donner une mousse qui est purifiée par chromatographie sur gel de silice (Si60, 20-45 µm ; MeOH (2 à 6 %)/$CH_2CL_2$). Les fractions concentrées cristallisent dans l'éther éthylique froid. M = 181 g ; Rdt = 70 % ; F = 257-266° C ; $[\alpha]_D^{22}$ = -103° (c = 0,6 ; $CHCl_3$).

**EXEMPLE 9 :**

**Solvate de l'étoposide 2, $CH_3CN$)**

Le produit issu de l'exemple 8 est dissous dans un minimum du mélange acétonitrile-eau 50/50 v/v, et laissé cristalliser lentement. Les cristaux obtenus sous forme de prismes maclés sont solvatés par une molécule de solvant. F = 183-186° C.

Un cristal est sélectionné en vue de l'étude structurale par diffraction des rayons X. L'échantillon est préparé sous forme d'un petit prisme taillé 0,15 x 0,18 x 0,20 mm. A titre de documentation ces données analytiques sont indiquées ci-après.

$C_{29}H_{32}O_{13}$, $CH_3CN$, : Mr = 629.6, monoclinique, $P2_1$, a = 11.997 (4), b = 6.111(2), c = 21.346(7) Å, $\beta$ = 97.69 (3)°, V = 1550.8(6) $A^{-3}$, Z = 2, $D_x$ = 1.35 Mg.$m^{-3}$, $\lambda(MOK\alpha)$ = 0.71069 Å, $\mu$ = 0.99 $cm^{-1}$, F(000) = 664, T = 296 K, R final = 0.051 pour 2153 observations.

L'étude a été réalisée à l'aide d'un diffractomètre automatique CAD4 ENRAF-NONIUS (anticathode en molybdène, monochromateur graphite) : Les paramètres de la maille ont été déterminés et affinés à partir d'un ensemble de 25 réflections à angle élevé. L'échantillon (petit prisme taillé à 0.15 x 0.18 x 0.20 mm) a fourni 3878 réflexions ($2\theta_{max}$ = 50°) dont 2153 indépendantes (Rint = 0.021) avec I> 1.2σ(I) : domaine analysé H (0-15) K (0-7) L(-27-27), balayageω/2 Θ, D, vitesse de balayage variable (t max = 60s), variation de 0.6 % (glissement non significatif) sur les contrôles d'intensité.

Après corrections de Lorenz et de polarisation, la structure a été résolue à l'aide des Méthodes Directes (Main, Fiske, Hull, Lessinger, Germain, Declercq and Woolfson, 1982) qui révèlent l'ensemble des atomes non-hydrogène du motif Etoposide. Après affinement du facteur d'échelle suivi d'une Différence de Fourier apparaît une molécule parasite identifiée comme étant de l'acétonitrile. L'ensemble du motif est alors affiné en mode isotrope (R = 0.393) puis en mode anisotrope (R = 0,078). A ce stade la position des atomes d'hydrogène est déterminée à l'aide d'une différence de Fourier (entre 0,36 et 0,18 e$Å^{-3}$).

Le meilleur affinement par matrice complète de l'ensemble de la molécule (x,y,z, βij) atomes non-hydrogène et x,y,z atomes d'hydrogène donne (2153 observations pour 406 variables ) :

R = 0,054

R = 0,051

S = 1,28

Δe = 0,12 e$Å^{-3}$.

$\omega = I/\sigma\text{-}(F_0)^2 = [\sigma^2(I) + (0{,}04\ F_0^2)^2]^{-1/2}$

Remarque :

Des calculs ont été également entrepris en vue de déterminer la configuration absolue de ce composé, les techniques actuelles ne nécessitant plus systématiquement la présence d'un atome lourd dans le motif. Il n'a pas été possible de trancher sans ambiguité entre les deux énantiomères, ce type de calcul exige en effet des cristaux d'excellente qualité ce qui n'était pas tout à fait le cas du fait vraisemblablement de la présence d'une molécule de solvant de cristallisation.

L'ensemble des calculs a été réalisé sur un mini-ordinateur DIGITAL PDP 11/60 à l'aide du jeu de programmes SDP (Frenz, 1985), les facteurs de diffusion sont issus des Tables Internationales de Cristallographie (1974), les vues en perspectives réalisées à l'aide du programme ORTEP (Johnson, 1965).

**REFERENCES**

FRENZ, B.A. (1985), ENRAF-NONIUS Structure Determination Package : SDP Users Guide, Version 1985. Collège Station, Texas 77840, USA, and Enraf-Nonius, Delft, The Netherlands.

International Tables for X-ray Crystallography (1974). Vol. IV. Birmingham : Kynoch Press. (Present

distributeur D. Reidel, Dordrecht).

JOHNSON, C.K. (1965). ORTEP. Report ORNL-3794. Oak Ridge National Laboratory Tennessee, USA.

MAIN, P., FISKE, S.J., HULLE, S.E., LESSINGER, L., GERMAIN, G., DECLERCQ, J.-P. & WOOLFSON, M.M. (1982). MULTAN 82. A System of Computer Programs for the Automatic Solution of Crystal Structures from X-ray Diffraction Data. Univs. of York, England, and Louvain, Belgium.

La figure annexée est une représentation spaciale de la molécule d'étoposide.

**EXEMPLE 10 :**

**Anomère alpha de l'étoposide I′**

L'anomère alpha de l'étoposide est la principale impureté issue du procédé de fabrication de l'étoposide. Il est séparé par chromatographie liquide industrielle à haute performance.

$$F=218\text{-}222°C.\ [\alpha]_D^{22}=+22°\ (c=1,\ CHCl_3).$$

**RMN¹H** 500 MHz ($CDCl_3$) (δppm) : 6,86 (1H, s, H-5) ; 6,50 (1H, s, H-8) ; 6,24 (2H, s, H-2′,6′) ; 5,97 et 5,96 (2H, 2d, $J_{AB}$ = 1,3 Hz, $OCH_2O$) ; 5,39 (1H, s, OH phénol) ; 5,04 (1H, d, $J_{1''\text{-}2''}$ = 4,0 Hz, H-1″) ; 4,7 (2H, m, H-4, 3a (A)) ; 4,67 (1H, q, $J_{7''\text{-}8''}$ = 5,1 Hz, H-7″) ; 4,59 (1H, d, $J_{1\text{-}2}$ = 5,3 Hz, H-1) ; 4,25 (1H, dd, H-3a (B)) ; 4,05 (1H, dd, $J_{AB}$ = 9,2 Hz, $J_{6''\text{-}5''}$ H-6″(A)) ; 3,82 (1H, t, H-3″) ; 3,75 (6H, s, $OCH_3$) ; 3,70 (1H, dd, $J_{2''\text{-}1''}$ = 4,0 Hz, $J_{2''\text{-}3''}$ = 9,3 Hz, H-2″) ; 3,51 (1H, t, H-6″ (B)) ; 3,48 (1H, m, H-5″) ; 3,36 (1H, dd, $J_{2-1}$ = 5,3 Hz, $J_{2-3}$ = 14,0 Hz, H-2) ; 3,25 (H-1, t, H-4″) ; 2,86 (1H, m, H-3) ; 2,72 (1H, s, OH) ; 2,60 (1H, s, OH) et 1,32 (3H, d, $J_{8''\text{-}7''}$ = 5,1 Hz, H-8″).

**Revendications**

**1.** Les tétra-O-acétyl-β-D-O-glucosides de déméthyl-4′ alcoxy-carbonyl-4′ épipodophyllotoxines répondant à la formule générale III

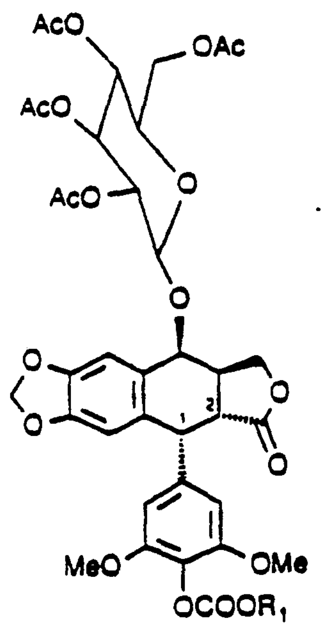

(III)

dans laquelle :

$R_1$ représente un radical alcoyle inférieur en $C_1$ à $C_4$, linéaire ou ramifié et éventuellement substitué par un ou plusieurs atomes d'halogène, en particulier de chlore.

**2.** Procédé de préparation d'un composé de formule générale III telle que définie à la revendication 1, caractérisé en ce que l'on met en oeuvre les étapes réactionnelles successives suivantes :

i) on protège sélectivement l'hydroxyde phénolique de la déméthyl-4′ épipodophyllotoxine de formule VII

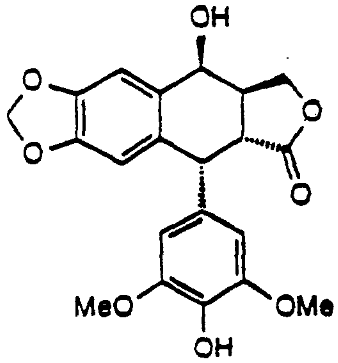

(VII)

par réaction de ce dernier composé de formule VII avec un chloroformiate d'alcoyle de formule VI
$ClCO_2$-$R_1$ (VI)
dans laquelle $R_1$ a la signification donnée à la revendication 1 à propos de la formule III, et
ii) on soumet l'alcoxycarbonyl-4' déméthyl-4' épipodophyllotoxine ainsi obtenue, qui répond à la formule générale V

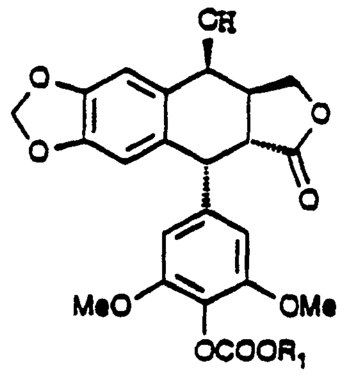

(V)

dans laquelle $R_1$ a la même signification que ci-dessus,
à une réaction de couplage avec le tétra-O-acétyl-2,3,4,6-β-D-glucose de formule IV

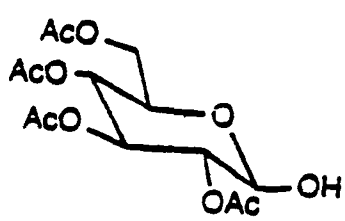

(IV)

**3.** Procédé selon la revendication 2, caractérisé en ce que la réaction de couplage ii) est réalisée en présence d'un acide de Lewis, dans un solvant de préférence constitué par un hydrocarbure chloré.

**4.** Application des composés de formule générale III selon la revendication 1 en tant qu'intermédiaires de synthèse utiles dans la préparation d'alkylidènes glucosides de déméthyl-4' épipodophyllotoxine.

**5.** Application selon la revendication 4, caractérisé en ce que l'on déprotège totalement un composé de formule III pour obtenir la β -D-glucopyranosyl-4 déméthyl-4' épipodophyllotoxine de formule II

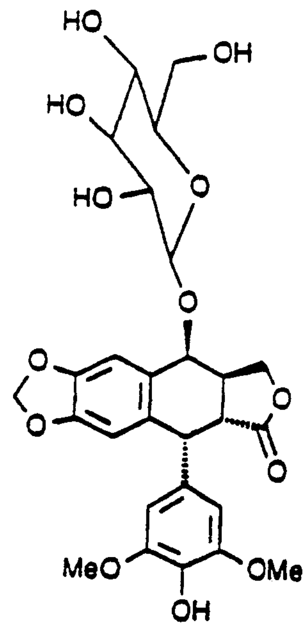

(II)

laquelle est ensuite soumise à une acétylation pour conduire à un alkylidène glucoside de déméthyl-4′ épipodophyllotoxine de formule générale I

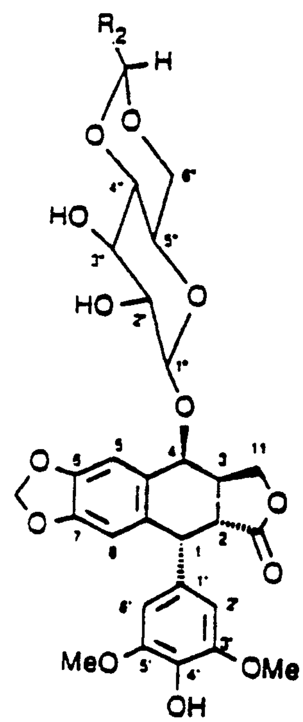

(I)

dans laquelle $R_2$ représente un radical alcoyle inférieur en $C_1$ à $C_4$, aryle ou hétéroaryle tel que thiophényle.

**6.** Application selon la revendication 5, caractérisée en ce que l'opération de déprotection totale du composé de formule III est réalisée en présence d'un mélange d'acétate métallique, notamment de zinc, et d'une suspension de poudre de zinc, à titre de catalyseur.

**7.** Application selon la revendication 5, caractérisée en ce que l'étoposide de formule I est soumis à une opération de purification par recristallisation dans l'acétonitrile aqueux.

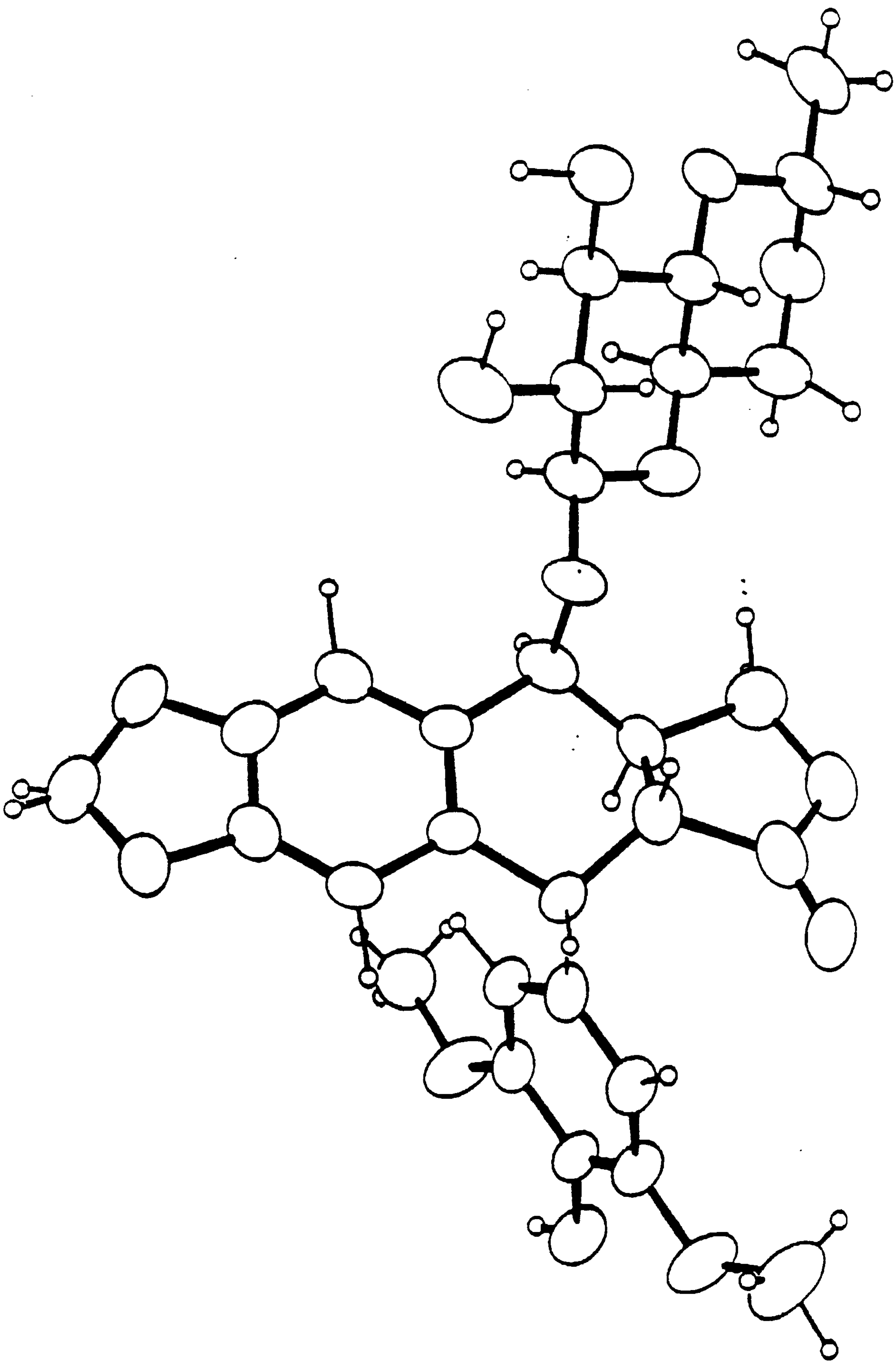

Office européen des brevets

# RAPPORT DE RECHERCHE EUROPEENNE

Numero de la demande

EP 90 40 3330

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-3 524 844 (C. KELLER-JUSLEN et al.)<br>* Colonne 1, ligne 71 - colonne 3, ligne 75; colonne 6, ligne 45 - colonne 7, ligne 37; colonne 23, lignes 34-75; colonne 24, ligne 59 - colonne 25, ligne 15 *<br>--- | 1-7 | C 07 H 17/04 |
| Y | CHEMICAL ABSTRACTS, vol. 100, no. 21, 21 mai 1984, page 670, résumé no. 175210d, Columbus, Ohio, US; & JP-A-58 219 196 (NIPPON KAYAKU CO., LTD) 20-12-1983<br>* Abrégé *<br>--- | 1-7 | |
| A | EP-A-0 226 202 (BAR-ILAN UNIVERSITY)<br>* Page 4, ligne 10 - page 5, ligne 10; page 11, lignes 1-16 *<br>--- | 1-7 | |
| A | EP-A-0 320 988 (BRISTOL-MEYERS)<br>* Abrégé; revendication 1 *<br>----- | 1-7 | |

| DOMAINES TECHNIQUES RECHERCHES (Int. Cl.5) |
|---|
| C 07 H 17/00 |

Le présent rapport a été établi pour toutes les revendications

| Lieu de la recherche | Date d'achèvement de la recherche | Examinateur |
|---|---|---|
| LA HAYE | 26-02-1991 | BRENNAN J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant